# EUROPÄISCHE PATENTANMELDUNG

(11) **EP 0 669 395 A2**
(43) Veröffentlichungstag der Anmeldung: **30.08.1995**
(21) Anmeldenummer: 95102669.9
(22) Anmeldetag: 24.02.1995
(51) Int. Cl.: C12N 15/10, C12N 9/12, C12Q 1/68

(54) **3'-RNA-Markierung mit Terminaler Transferase**

(30) Priorität: 28.02.1994 DE 4406524
(71) Anmelder: BOEHRINGER MANNHEIM GMBH, D-68305 Mannheim-Waldhof (DE)
(72) Erfinder: Seibl, Rudolf, Dr., D-82377 Penzberg (DE); Laubrock, Andreas, D-24106 Kiel (DE); Rosemeyer, Viola, B-1300 Wavre (BE)
(74) Vertreter: Weiss, Wolfgang, Dipl.-Chem. Dr.

(57) **Zusammenfassung**

Die vorliegende Erfindung betrifft ein Verfahren zum Einführen von nicht-radioaktiv markierten Desoxynucleotiden in Nucleinsäuren sowie RNA-Moleküle, die an ihrem 3'-Ende mindestens ein Desoxynucleotid enthalten, das eine nicht-radioaktive Markierungsgruppe trägt.

### SEQUENZPROTOKOLL

(1) ALLGEMEINE ANGABEN:
   (i) ANMELDER:
      (A) NAME: Boehringer Mannheim
      (B) STRASSE: Sandhofer Str. 112-132
      (C) ORT: Mannheim-Waldorf
      (E) LAND: Bundesrepublik Deutschland
      (F) POSTLEITZAHL: 68305
      (G) TELEFON: (0621)759-3277
      (H) TELEFAX: (0621)759-4457
   (ii) BEZEICHNUNG DER ERFINDUNG: 3'-RNA Markierung mit Terminaler Transfer
   (iii) ANZAHL DER SEQUENZEN: 4
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
   (vi) DATEN DER URANMELDUNG:
      (A) ANMELDENUMMER: DE P 44 06 524.8
      (B) ANMELDETAG: 28-FEB-1994
(2) ANGABEN ZU SEQ ID NO: 1:
   (i) SEQUENZKENNZEICHEN:
      (A) LÄNGE: 25 Basenpaare
      (B) ART: Nucleotid
      (C) STRANGFORM: Einzelstrang
      (D) TOPOLOGIE: linear
(xi) SEQUENZBESCHREIBUNG: SEQ ID NO: 1:
   CGCCGCGUCG CAGAAGAUCU CAAUC 25
(2) ANGABEN ZU SEQ ID NO: 2:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 2:
   TTGAGATCTT CTGCGACGCG G 21
(2) ANGABEN ZU SEQ ID NO: 3:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
   TTGAGATCTT ATGCGACGCG G 21
(2) ANGABEN ZU SEQ ID NO: 4:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
   TTGAGATCTC ACGCGACGCG G 21

## Beschreibung

Die vorliegende Erfindung betrifft ein Verfahren zum Einführen von nicht-radioaktiv markierten Desoxynucleotiden in Nucleinsäuren sowie RNA-Moleküle, die an ihrem 3'-Ende mindestens ein Desoxynucleotid enthalten, das eine nicht-radioaktive Markierungsgruppe trägt.

Markierte RNA-Moleküle werden oft als Hybridisierungssonden verwendet, da RNA-DNA-Hybride stabiler als DNA-DNA-Hybride sind (Srivastava, R.A.K. und Schonfeld, G. (1991) BioTechniques 11, 584-587). Eine wohlbekannte Methode zur Herstellung von nicht-radioaktiv markierten RNA-Sonden ist die in vitro Transkription unter Verwendung von markierten Nucleotiden als Substrate für die RNA-Polymerasen (Langer, P.R., Waldrop, A.A. und Ward, D.C. (1981) Proc.Natl.Acad.Sci. USA 78, 6633-6637; Höltke, H.-J. und Kessler, C. (1990) Nucleic Acids Res. 18, 5843-5851). Bei einer in vitro Transkription kann RNA am 5'-Ende unter Verwendung biotinylierter Dinucleotide als Initiator-Oligonucleotide markiert werden (Pitulle, C., Kleineidam, R.G., Sproat, B. und Krupp, G. (1992) Gene 112, 101-105).

Bereits vorhandene RNA-Moleküle können dagegen nur durch Verwendung von Polynucleotidkinase und [y³²P]-ATP,d.h. radioaktiv, mit einer enzymatischen 5'-Endmarkierung versehen werden (Richardson, C.C. (1981) In Boyer, P.D. (ed.) The Enzymes. Academic Press, New York, Vol. XIV, pp. 299-314).

Eine chemische 3'-Endmarkierung von RNA-Molekülen mit Biotin wird von Broker et al. (Nucleic Acids Res. 5 (1978), 363-384) und Sodja und Davidson (Nucleic Acids Res. 5 (1978), 385-401) beschrieben. Biotin wird dabei in einer mehrstufigen Reaktion über einen NH₂(CHs)NH₂-Spacer oder über Cytochrom c an den 3'-terminalen Ribosezucker der RNA gebunden. Diese Reaktion umfaßt die Oxidation des Zuckers mit Perjodat, die Reaktion des resultierenden Dialdehyds mit einer NH₂-Gruppe des Spacers oder des Cytochrom c unter Bildung einer Schiff-Base, eine anschließende Reduktion mit BH₄- und die kovalente Kopplung von Biotin an den Spacer oder das Cytochrom c. Dieses Markierungsverfahren ist ein langwieriger und aufwendiger Prozeß, der in der Praxis keine Anwendung gefunden hat.

Eine radioaktive enzymatische 3'-Endmarkierung von RNA wurde mit Poly(A)-Polymerase und T4 RNA-Ligase gezeigt. Poly(A)-Polymerase wird zum Anfügen von [a³²P]-ATP an das 3'-Ende von RNA-Molekülen verwendet (Winter, G. und Brownlee, G.G. (1978) Nucleic Acids Res. 5, 3129-3139). T4 RNA-Ligase wird zum Anfügen von [5'-³²P]-pCp an das 3'-Ende von RNA-Molekülen verwendet (Uhlenbeck, O.C. und Gumport, R.I. (1982) In Boyer, P.D. (ed.) The Enzymes, Academic Press, New York, Vol XV, S. 31-58). T4 RNA-Ligase kann auch zur nicht-radioaktiven Markierung von RNA mit Biotin-, Tetramethylrhodamin- und Fluoresceinderivaten von P'-(6-Amino-hex-1-yl)-P²⁻(5'-adenosin)pyrophosphat verwendet werden (Richardson, R.W. und Gumport, R.I. (1983) Nucleic Acids Res. 11, 6167-6184).

Das Anfügen von Nucleotiden an das 3'-Ende von RNA-Molekülen mit RNA-Ligase und Poly(A)-Polymerase weist jedoch erhebliche Schwierigkeiten auf. Bei der RNA-Ligase bestehen prinzipiell zwei Möglichkeiten der 3'-Markierung von RNA durch Verwendung verschiedener Arten von Substraten, nämlich der Markierung mit einem einzigen Nucleotid und der Markierung durch Ligation mit einem 3'-markierten Oligonucleotid. Bei Markierung mit einem einzelnen Nucleotid muß dieses Nucleotid an 3'- und 5'-Position ein Phosphat aufweisen, d.h. es muß in der Form pNp vorliegen. Die Anfügung eines derartigen Nucleotids, das eine nichtradioaktive Markierungsgruppe trägt, an ein RNA-Molekül mit Hilfe der T4 RNA-Ligase ist nicht bekannt. Nicht-radioaktive Markierungsgruppen können mit der T4 RNA-Ligase daher nur direkt mit einem Spacer auf das 3'-Hydroxylende von RNA übertragen werden (vgl. Richardson und Gumport, supra). Die Anfügung von Nucleotiden mit einer an die Nucleotidbase gebundenen Markierungsgruppe an das 3'-Ende von RNA-Molekülen ist nicht bekannt.

Weiterhin sind bei Verwendung eines einzelnen Nucleotids als Substrat keine Variationen in der Anzahl der angefügten Nucleotide und damit in der Intensität der Markierung möglich, da pro Reaktionszyklus immer nur ein einziges Substratmolekül angefügt werden kann. Bei Verwendung eines 3'-markierten Oligonucleotids als Substrat für die T4 RNA-Ligase wird eine zusätzliche Sequenz mit angehängt, die z.B. bei Hybridisierungsversuchen stören könnte. Überdies sind zum effizienten Anfügen von Substratmolekülen an RNA durch die T4 RNA-Ligase lange Inkubationszeiten von beispielsweise ca. 12 Stunden erforderlich, was zu einem signifikanten Abbau der stark hydrolyseempfindlichen RNA führen kann.

Die effiziente Markierung der 3'-Enden von RNA-Molekülen mit Poly(A)-Polymerase ist auf die Verwendung von ATP und ATP-Derivaten beschränkt, da andere Basen als A vom Enzym viel schlechter akzeptiert werden. Oligonukleotide weisen als Akzeptormoleküle eine äußerst geringe Effizienz auf. Das Anfügen von Oligoribonucleotiden an das 3'-Ende von RNA-Molekülen durch die Poly(A)-Polymerase ist nicht bekannt. Eine Möglichkeit der Anfügung nicht-radioaktiv markierter Nucleotide besteht nicht.

In einer Arbeit von Roychoudhury und Kössel (Eur. J. Biochem. 22 (1971), 310-320) wird beschrieben, daß ein Oligodesoxynucleotid mit zwei Ribonucleotideinheiten am 3'-Ende als Nucleinsäure-Akzeptormolekül zum Anfügen von dATP-Molekülen durch die Terminale Desoxynucleotidyltransferase dienen kann. Durch die Anwesenheit der zwei Ribonucleotide am 3'-Ende des Nucleinsäure-Akzeptormoleküls wird jedoch selbst bei sehr hohen ATP-Konzentrationen die Reaktionsgeschwindigkeit extrem verringert.

In einer Arbeit von Feix (FEBS Letters 18 (1971), 280-282) wird beschrieben, daß das Oligoribonucleotid A₆ als Nucleinsäure-Akzeptormolekül zum Anfügen von dCTP an das 3'-Ende durch die Terminale Desoxynucleotidyltransferase dienen kann. Es wurde jedoch gefunden, daß der Einbau von dCTP in das Ribonucleinsäure-Akzeptormolekülmit einer äußerst geringen Effizienz verglichen mit einem Desoxyribonucleinsäure-Akzeptormolekül erfolgt. In einer späteren Arbeit schreibt der gleiche Autor (Feix, Biochem. Biophys. Res. Comm. 46 (1972), 2141-2147), daß ein Ribonucleinsäure-Akzeptormolekül als Primer für das Anfügen von Desoxynucleotiden durch die Terminale Desoxynucleotidyltransferase eine vernachlässigbare Aktivität zeigt.

Aus dem Stand der Technik ist daher kein Verfahren bekannt, mit dem bereits vorhandene RNA-Moleküle auf einfache Weise mit einer oder mehreren nicht-radioaktiven Markierungsgruppen versehen werden können.

Die der vorliegenden Erfindung zugrundeliegende Aufgabe bestand somit darin, ein Verfahren zum Einführen von nicht-radioaktiven Markierungsgruppen in RNA-Moleküle bereitzustellen, bei dem die Nachteile und Schwierigkeiten des Standes der Technik mindestens teilweise beseitigt sind. Insbesondere bestand die Aufgabe der vorliegenden Erfindung darin, ein Verfahren bereitzustellen, mit dem eine effiziente, schnelle Einführung von unterschiedlichen Nucleotiden, die nicht-radioaktive Markierungsgruppen tragen, in RNA-Moleküle möglich ist.

Diese Aufgabe wird gelöst durch ein Verfahren zum Einführen von Nucleotiden, die eine nicht- radioaktive Markierungsgruppe tragen, in Nucleinsäuren, welches dadurch gekennzeichnet ist, daß man mindestens ein nicht-radioaktiv markiertes Desoxynucleotid an das 3'-Ende eines Nucleinsäure-Akzeptormoleküls mit mindestens einem 3'-terminalen Ribonucleotid durch die Terminale Desoxy/nucleotidyltransferase (EC 2.7.7.31) anfügt.

Durch das erfindungsgemäße Verfahren ist es überraschenderweise möglich, nicht-radioaktive Markierungsgruppen durch eine einfache enzymatische Reaktion innerhalb einer kurzen Zeitdauer an das 3'-Ende eines Nucleinsäure-Akzeptormoleküls mit einem 3'-terminalen Ribonucleotid anzufügen. Die Reaktion verläuft effizient und es ist möglich, in einem einzigen Reaktionszyklus mehrere Markierungsgruppen, die gegebenenfalls auch verschieden sein können, in das Nucleinsäure-Akzeptormolekül einzuführen. Ein weiterer Vorteil des Verfahrens ist, daß die Reagenzien, d.h. sowohl die Terminale Desoxynucleotidyltransferase als auch Desoxynucleotide mit nicht-radioaktiver Markierungsgruppe kommerziell erhältliche Substanzen sind.

Die Terminale Desoxynucleotidyltransferase (auch Nucleosidtriphosphat: DNA Desoxynucleotidylexotransferase oder TdT) ist ein Enzym, das im Knochenmark und Thymus von Säugern vorkommt (siehe z.B. Kung, P.C., Gottlieb, P.D. und Baltimore, D., J.Biol.Chem. 251 (1976), 2399-2404; Ratcliff, R.L. ,The Enzymes 14a (1981), 105-118) und mit einer spezifischen Aktivität von ca. 60000 U/mg z.B. von Boehringer Mannheim GmbH, Mannheim, BRD, erhältlich ist. Für das erfindungsgemäße Verfahren ist sowohl aus Säugergewebe (insbesondere Kalbsthymus) isolierte TdT als auch durch rekombinante DNA-Techniken hergestellte TdT geeignet.

Die Verwendung von Terminaler Desoxynucleotidyltransferase (TdT) zum Anfügen von einem oder mehreren Desoxynucleotiden an das 3'-Ende von einzelsträngiger oder doppelsträngiger DNA und zur Markierung von Oligodeoxyribonucleotiden mit Biotin- oder Digoxigenin-modifizierten Nucleotiden ist bekannt (siehe z.B. Deng, G. R. und Wu, R. (1983) Methods Enzymol. 100, 96-116; Hayes, F.N., Mitchell, V.E., Ratliff, R.L., Schwartz, A.W. und Williams, D.L. (1966) Biochemistry 5, 3625-3629; Roychoudhury, R., Jay, E. und Wu, R. (1976) Nucleic Acids Res. 3, 863-877; Kumar, A., Tchen, P., Roullet, F. und Cohen, J. (1988) Anal. Biochem. 169, 376-382; Riley, L.K., Marshall, M.E. und Coleman, M.S. (1986) DNA 5, 333-337; Schmitz, G.G., Walter, T., Seibl, R. and Kessler, C. (1991) Anal. Biochem. 192, 222-231). Desoxynucleotide mit einer nicht-radioaktiven Markierungsgruppe werden von der TdT jedoch wesentlich schlechter als unmarkierte oder radioaktiv markierte Desoxynucleotide akzeptiert.

Daher war es im höchsten Maße überraschend, daß Desoxynucleotide, die eine nicht-radioaktive Markierungsgruppe tragen, überhaupt als Substrate der TdT akzeptiert werden, wenn man ein Nucleinsäure-Akzeptormolekül mit mindestens einem 3'-terminalen Ribonucleotid verwendet.

Das Nucleinsäure-Akzeptormolekül oder der Primer im erfindungsgemäßen Verfahren ist eine Nucleinsäure, an welche die Substratmoleküle, d.h. im allgemeinen Desoxyribonucleotidtriphosphate, unter Abspaltung von Pyrophosphat und Bildung einer Phosphodiesterbindung angefügt werden. Diese Nucleinsäure weist an ihrem 3'-Ende mindestens einen Ribosezucker mit einer freien 3'-Hydroxylgruppe auf und kann jede beliebige chemisch oder/und enzymatisch synthetisierte Nucleinsäure, z.B. eine in vivo von einer Zelle erzeugte Nucleinsäure sein, die abgesehen von dem 3'-terminalen Ribonucleotid beliebige Nucleotideinheiten, d.h. insbesondere Desoxyribonucleotid- oder/und Ribonucleotideinheiten enthalten kann. Bevorzugt sind jedoch Nucleinsäure-Akzeptormoleküle, die an ihrem 3'-Ende mindestens zwei und insbesondere mindestens drei Ribosezucker aufweisen. Besonders bevorzugt sind Nucleinsäuremoleküle, die zu mehr als 50 % und im wesentlichen ausschließlich aus Ribonucleotideinheiten aufgebaut sind, d.h. RNA-Moleküle, insbesondere solche, die in vivo erzeugt worden sind.

Die Mindestlänge des Nucleinsäure-Akzeptormoleküls für das erfindungsgemäße Verfahren beträgt 3, vorzugsweise 8 Nucleotide und besonders bevorzugt 12 Nucleotide. Die maximale Länge des Nucleinsäure-Akzeptormoleküls ist an sich beliebig, d.h. es können ohne weiteres mehrere Tausend, z.B. 2000 bis 3000 Basen lange Nucleinsäuren eingesetzt werden. Weiterhin ist es bevorzugt, daß das Nucleinsäure-Akzeptormolekül im erfindungsgemäßen Verfahren in Form eines einzelsträngigen Moleküls und ohne stabile, ausgeprägte Sekundärstrukturen (z.B. Haarnadelstrukturen) an seinem 3'-Ende vorliegt. Beispielsweise können für das erfindungsgemäße Verfahren auch mRNA-Moleküle als Nucleinsäure-Akzeptormoleküle eingesetzt werden.

Im erfindungsgemäßen Verfahren werden Desoxynucleotide und zwar insbesondere 2'-Desoxynucleosidtriphosphate oder/und 2',3'-Desoxynucleosidtriphosphate als Substrate an eine Nucleinsäure angefügt. Bei Verwendung von 2'-Desoxynucleosidtriphosphaten als Substrate kann das 3'-Ende der Akzeptor- Nucleinsäure um mehrere Nucleotide verlängert werden, während die Anfügung eines 2',3'-Desoxynucleosidtriphosphats zu einem 3'-H-Ende und somit zu einem Kettenabbruch führt.

Die Nucleotidbasen in den nicht-radioaktiv markierten Desoxynucleotiden können die natürlichen Basen Adenin, Guanin, Cytosin, Thymin, Uracil sowie Basenanaloga wie etwa Inosin, Pseudouracil, Fluoruracil und dgl. sein. Spezifische Beispiele der Desoxynucleotide sind etwa Biotin-dUTP und Digoxigenin-dUTP.

Bei dem erfindungsgemäßen Verfahren können als Substrate einerseits nicht-radioaktiv markierte Desoxynucleotide alleine oder Kombinationen von unmarkierten (oder z.B. radioaktiv markierten) Desoxynucleotiden und nicht-radioaktiv markierten Desoxynucleotiden eingesetzt werden. Die Verwendung von Kombinationen aus unmarkierten und markierten Desoxyribonucleotiden ist bevorzugt, wenn mehrere Markierungsgruppen in die Akzeptor-Nucleinsäure eingeführt werden sollen.

Durch das erfindungsgemäße Verfahren wird auch eine neue und wirksame Methode zur enzymatischen Einführung von Markierungsgruppen in verschiedene Typen von RNA-Molekülen durch Anfügung von vorzugsweise nicht-radioaktiv markierten Desoxynucleotiden an das 3'-Ende der RNA-Moleküle bereitgestellt. Vorzugsweise verwendet man als RNA-Moleküle eine Population von in vivo erzeugten RNA-Molekülen, z.B. mRNA-Moleküle, die aus einer Zelle isoliert wurden.

Bevorzugte Beispiele für Nucleotide, die eine nicht-radioaktive Markierungsgruppe tragen, sind Nucleotide, die eine Fluoreszenz-, Chemilumineszenz-, NMR-aktive oder/und zur hochaffinen Bindung mit einem Reaktionspartner fähige Markierungsgruppe tragen. Besonders bevorzugt sind Markierungsgruppen, die zur hochaffinen Bindung mit einem Reaktionspartner fähig sind, z.B. Biotin oder Biotinderivate, die zur Bindung mit Avidin oder Streptavidin fähig sind, oder Haptene, die zur Bindung mit einem spezifischen Antikörper fähig sind. Besonders bevorzugt sind Digoxigenin- oder Biotin-modifizierte Nucleotide. Es können jedoch auch andere nicht-radioaktiv markierte Desoxynucleotide, wie etwa mit Fluorescein, Rhodamin, Coumarin, anderen Fluorophoren oder sonstigen Haptenen modifizierte Desoxynucleotide verwendet werden. Weitere Beispiele für geeignete Markierungsgruppen finden sich in "Nonradioactive Labeling and Detection of Biomolecules, C. Kessler ed., Springer Verlag 1992, insbesondere S.6; S. 28).

TdT kann zum Anfügen eines einzigen modifizierten 2',3'-Didesoxynucleotids an das 3'-Ende eines Nucleinsäure-Akzeptormoleküls verwendet werden. Andererseits können auch mehrere Desoxynucleotide an derartige RNA-Moleküle unter Verwendung von einem oder mehreren verschiedenen modifizierten 2'-Desoxynucleosidtriphosphaten, z.B. Digoxigenin-dUTP oder Biotin-dUTP angefügt werden. Die Anfügung einer größeren Anzahl von Desoxynucleotiden kann beispielsweise mit einer Mischung von unmarkierten und markierten 2'-Desoxynucleotidtriphosphaten, z.B. Digoxigenin-dUTP und dATP, erfolgen.

Durch das erfindungsgemäße Verfahren können unter Verwendung geeigneter Reaktionsbedingungen bei der 3'-Endmarkierung von Nucleinsäure-Akzeptormolekülen mit Desoxynucleotiden, die eine nicht- radioaktive Markierungsgruppe tragen, in einem einzigen Reaktionszyklus mehrere Markierungen in ein einziges Nucleinsäure-Akzeptormoleküleingeführt werden. Vorzugsweise werden bis zu 20 Markierungen eingefügt, wodurch der Nachweis der markierten Nucleinsäure mit einer sehr hohen Sensitivität ermöglicht wird. Besonders bevorzugt werden 2 bis 10 Markierungsgruppen eingefügt. Die Markierungsgruppen können gleich oder verschieden sein. Bevorzugt werden insgesamt bis zu 200 (unmarkierte und markierte) Desoxynucleotide pro Reaktionszyklus, besonders bevorzugt 1 bis 50 Desoxynucleotide pro Reaktionszyklus angefügt.

Die Konzentration des Nucleinsäure-Akzeptormoleküls im Reaktionsansatz beträgt vorzugsweise 0,1 umol/1 bis 20 µrnol/l, besonders bevorzugt von 0,5 umol/1 bis 10 µmol/l. Die Konzentration der Deoxynucleotide beträgt vorzugsweise 0,1 bis 2,0 mmol/I im Reaktionsansatz. Wenn ein einziges Nucleotid an das 3'-Ende der Akzeptornucleinsäure angefügt werden soll, verwendet man als Substrat ein 2',3'-Didesoxynucleotid, dessen Anfügung zu einem Kettenabbruch führt. Zur Anfügung einer geringeren Anzahl von Nucleotiden verwendet man als Substrat ein oder mehrere markierte 2'-Desoxynucleotide. Zur Anfügung einer größeren Anzahl (z.B. > 10) von Nucleotiden kann man als Substrat ein Gemisch aus einem oder mehreren unmarkierten 2'-Desoxynucleotiden und einem oder mehreren markierten 2'-Desoxynucleotiden verwenden, wobei das Mol-Verhältnis von unmarkierten zu markierten Substratmolekülen im Bereich von vorzugsweise 2:1 bis 20:1, besonders bevorzugt 5:1 bis 15:1 ist.

Ein besonderer Vorteil des erfindungsgemäßen Verfahrens gegenüber bekannten RNA-Endmarkierungsverfahren besteht darin, daß die Reaktion innerhalb einer kurzen Dauer von vorzugsweise 10 min bis 2 Stunden, besonders bevorzugt von 20 min bis 1 Stunde, am meisten bevorzugt ca. 30 min durchgeführt werden kann. Weiterhin ist bevorzugt, daß die Reaktion in Anwesenheit eines RNase-Inhibitors, z.B. RNAsin durchgeführt wird, da kommerzielle TdT-Präparate eine gewisse RNase-Restaktivität enthalten können.

Andererseits ist es bevorzugt, eine ein im wesentlichen RNasefreie TdT zu verwenden. Die Entfernung der RNase-Restaktivität aus TdT-Präparationen kann auf an sich bekannte Weise (siehe z.B. Sambrook et al., Molecular Cloning. A Laboratory Manual, 2nd edition (1989), Cold Spring Harbor Laboratory Press, USA, S. B17-B19) durch Affinitätschromatographie an Agarose-5'-(4-aminophenylphosphoryl)-uridin-2'-(3')-phosphat, durch Adsorption an Macaloid oder durch Erhitzen in Gegenwart von Jodacetat erfolgen. Zur Durchführung des erfindungsgemäßen Verfahrens ist eine TdT besonders bevorzugt, die in einem Funktionstest, z.B. bei Inkubation von 4 µg MS2-RNA für 4 h bei 37°C in 50 µl Testpuffer mit TdT, keine meßbare Veränderung der RNA-Bande bei einer Konzentration von bis zu 50 U/ml, vorzugsweise von bis zu 500 U/ml TdT zeigt.

Weiterhin ist bevorzugt, daß die Reaktion bei einem pH-Wert von 6,0 bis 7,5 und bei einer Konzentration eines zweiwertigen Metallions, insbesondere Zn²⁺, Co²⁺, Mg²⁺ oder Mn²⁺ von 2 bis 10 mmol/I durchgeführt wird.

Besonders bevorzugte Bedingungen für einen Reaktionsansatz sind 20 - 200 pmol Nucleinsäure-Akzeptormolekül, 5 - 50 U TdT (1 U TdT ist die Enzymaktivität, die in 60 min bei 37°C mit (dT)₆ als Nucleinsäure-Akzeptor den Einbau von 1 nmol dAMP in säureunlösliche Produkte bewirkt), 100 - 300 mmol/I Kaliumcacodylat, 5 - 50 mmol/I Tris/HCI pH 6,6, 0,05 -0,5 mg/ml Rinderserumalbumin, 2 - 10 mmol/I CoCl₂ und 0,1 - 2,0 mmol/I Desoxynucleotid in einem Endvolumen von 10 - 200 µl. Die Reaktion wird etwa für 30 Minuten bei 37°C durchgeführt. Die Anfügung eines einzigen Nucleotids an das 3'-Ende kann z.B. durch Verwendung von 0,5 mmol/I eines 2',3'-Didesoxynucleotids, z.B. Digoxigenin-11-ddUTP oder Biotin-16-ddUTP, erreicht werden. Zur Anfügung einer geringeren Anzahl (z.B. bis zu 5) von Nucleotiden kann man 0,5 mmol/I eines markierten 2'-Desoxynucleotids, z.B. Digoxigenin-11-dUTP oder Biotin-16-dUTP oder eine Kombination von Digoxigenin-11-dUTP und Digoxigenin-11-ddUTP (jeweils 0,25 mmol/I) verwenden. Zur Anfügung einer größeren Anzahl (z.B. > 10) von Nucleotiden kann man beispielsweise ein Gemisch verwenden, das 0,45 mmol/I eines nicht-markierten 2'-Desoxynucleotids, wie etwa dATP, und 0,05 mmol/I eines markierten 2'-Desoxynucleotids enthält, z.B. Digoxigenin-11-dUTP, enthält. Weiterhin ist es bevorzugt, dem Ansatz ca. 40 U RNase-Inhibitor zuzusetzen oder TdT ohne meßbare RNase-Aktivität zu verwenden. Die Reaktion kann durch Zugabe von EDTA gestoppt werden.

In einer weiteren bevorzugten Ausführungsform der vorliegenden Erfindung kann man ein bereits markiertes Nucleinsäure-Akzeptormolekül als Ausgangsmaterial verwenden, z.B. ein am 5'-Ende markiertes Akzeptormolekül. Die Einführung einer Markierung am 5'-Ende einer Ribonucleinsäure kann im Falle einer radioaktiven Markierung durch Polynucleotidkinase und [y³²P]-ATP oder im Falle einer nicht-radioaktiven Markierung durch chemische Synthese, z.B. unter Verwendung eines entsprechenden modifizierten Phosphoramidits, z.B. eines Biotin-Amidits (Applied Biosystems) oder durch Einbau von Aminolink II (Applied Biosystems) und anschließende Kopplung mit der Markierung (G.H. Keller und M.H. Manak, DNA Probes, Stockton Press. 1989, Seite 136 ff.) erfolgen. Auf diese Weise ist es möglich, RNA-Moleküle herzustellen, die sowohl am 5'-Ende als auch am 3'-Ende eine Markierung tragen. Insbesondere ist die Herstellung von Molekülen mit zwei unterschiedlichen nicht-radioaktiven Markierungen am 5'- und am 3'-Ende bevorzugt, z.B. RNA-Moleküle mit einem Biotinrest am 5'-Ende und einem oder mehreren Digoxigemin-Resten am 3'-Ende oder umgekehrt.

Ein weiterer besonderer Vorteil des erfindungsgemäßen Verfahrens besteht darin, daß die terminale Desoxynucleotidyltransferase im Gegensatz etwa zu Poly(A)-Polymerase alle Nucleotide (z.B. dATP, dGTP, dCTP, dTTP, dUTP, dITP) und die entsprechende 2',3'-Didesoxyanaloga als Substrate akzeptiert.

Ein weiterer Gegenstand der vorliegenden Erfindung ist ein RNA-Molekül, das im Bereich seines 3'-Endes mindestens ein Desoxynucleotid enthält, das eine nicht-radioaktive Markierungsgruppe trägt, wobei die Markierung kovalent an die Nucleotidbase des Desoxynucleotids gebunden ist. Derartige Moleküle können durch Verwendung von bekannten enzymatischen RNA-Markierungsmethoden, z.B. durch Markierung mit Poly(A)-Polymerase oder T4 RNA-Ligase, nicht hergestellt werden. Bei der von Richardson und Gumport, supra, beschriebenen Methode der nicht-radioaktiven Markierung von RNA mit RNA-Ligase wird ein ADP eingesetzt, an dessen ß-Phosphoratom ein Aminohexylrest mit der nicht-radioaktiven Markierung gebunden ist. Die RNA-Ligase überträgt diesen Aminohexylrest unter Freisetzung von AMP auf das 3'-Hydroxylende einer RNA. Als Ergebnis ist die Markierung über den Aminohexylspacer endständig an einem Phosphatrest gebunden. Im Gegensatz dazu ist die durch das erfindungsgemäße Verfahren angebrachte Markierung kovalent an die Nucleotidbase des angefügten Nukleotids gebunden. Der Vorteil des erfindungsgemäßen Verfahrens ist dabei, daß das 3'-Ende der RNA noch zur Verfügung steht - entweder zur Anligierung einer zweiten Sequenz oder zum Anfügen von weiteren Nucleotiden durch die Terminale Transferase, d.h. es können mehrere Markierungsgruppen eingeführt werden, was zu einer Erhöhung der Sensitivität führt. Vorzugsweise trägt das erfindungsgemäße RNA-Molekül mindestens 2 nicht-radioaktive Markierungsgruppen, am meisten bevorzugt 2 - 10 Markierungsgruppen.

Vorzugsweise ist das nicht-radioaktiv markierte Desoxynucleotid im erfindungsgemäßen RNA-Molekül ein Digoxigenin- oder ein Biotin-markiertes Desoxynucleotid. Weiterhin ist bevorzugt, daß das RNA-Molekül zusätzlich eine Markierung im Bereich seines 5'-Endes trägt.

Ein weiterer Gegenstand der vorliegenden Erfindung ist die Verwendung der Produkte des erfindungsgemäßen Verfahrens im Rahmen der molekularbiologischen Forschung und der Pharmazeutik, z.B. als Sonde zum Nachweis von Nukleinsäureanalyten, als Template zur Herstellung von cDNA-Molekülen, bei der Isolierung und Reinigung von Nucleinsäuren, bei der Sequenzierung von RNA-Molekülen sowie zur Herstellung von Antisense-Nucleinsäuren.

Eine bevorzugte Anwendung ist der Nachweis eines Nucleinsäureanalyten mit einer definierten Zielsequenz in einer Probeflüssigkeit. Die Spezifität des Nachweises von Nucleinsäuremolekülen wird durch Hybridisierung mit einer zur Zielsequenz komplementären Nucleinsäuresequenz erreicht. Die direkte oder indirekte Identifizierung solcher Hybride läßt somit auf die Anwesenheit der Zielsequenz schließen. Ein Problem bei diesen Nachweisverfahren ist die oft geringe Menge an Ziel-DNA. Um dennoch ein deutliches, sich vom Hintergrund abhebendes Signal zu erhalten, können unterschiedliche Amplifikationstechniken angewandt werden: Die Amplifikation der Zielsequenz, die Amplifikation des Signals oder die Kombination beider Amplifikationsarten.

Eine Möglichkeit der Signalamplifikation ist das von P. Duck et al. in BioTechniques 9 (1990), 142-147 beschriebene "Cycling Probe"-Verfahren, das die Schritte umfaßt:
(a) Inkontaktbringen einer Probeflüssigkeit mit zu einem Abschnitt eines DNA-Analyten komplementären, markierten RNA-Molekülen unter solchen Bedingungen, daß bei Anwesenheit des Analyten eine Hybridisierung mit den markierten RNA-Molekülen stattfindet,
(b) Behandeln der Probeflüssigkeit mit RNase H unter solchen Bedingungen, daß bei Vorhandensein eines RNA-DNA-Hybrids ein Abbau der RNA-Moleküle im Hybrid und eine Freisetzung der abgebauten RNA-Bruchstücke stattfindet,
(c) gegebenenfalls ein- oder mehrmaliges Wiederholen der Schritte (a) und (b) und
(d) Nachweisen des Vorhandenseins oder des Fehlens des DNA-Analyten durch Bestimmung der abgebauten RNA-Bruchstücke oder/und der nicht abgebauten RNA-Moleküle.

Dieses Cycling-Probe Verfahren ist im folgenden schematisch dargestellt:

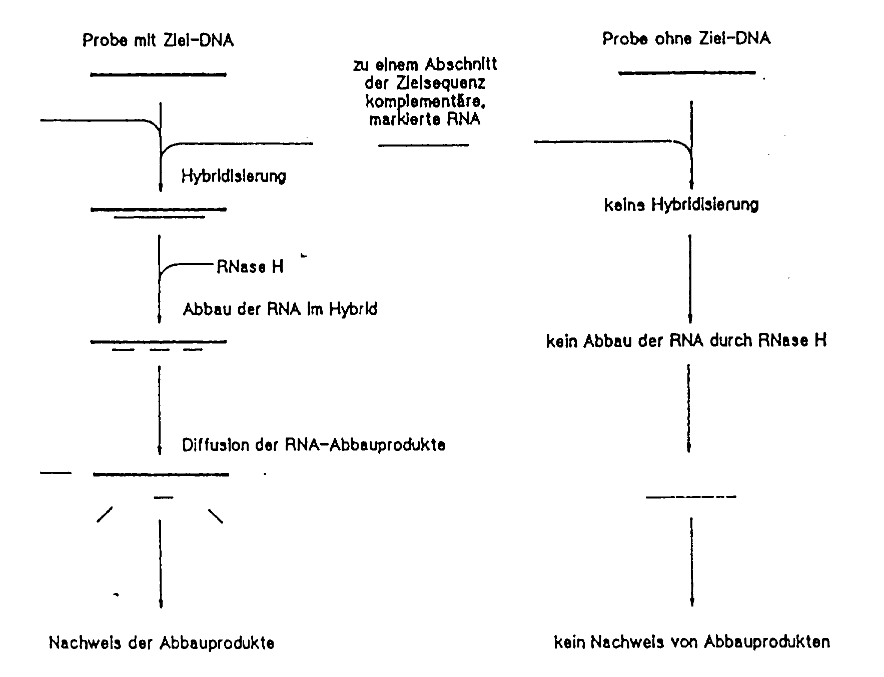

Zu einem bestimmten Abschnitt der Zielsequenz komplementäre RNA-Moleküle bilden mit der nachzuweisenden DNA RNA-DNA-Hybride aus. Durch das Einwirken von RNase H wird in diesen Hybriden jeweils nur der RNA-Anteil abgebaut und die Bruchstücke diffundieren aufgrund der geringeren Schmelztemperatur von der Zielsequenz ab. Dadurch steht die DNA für einen erneuten Hybridisierungs- und Abbauzyklus zur Verfügung. Sind die RNA-Moleküle im molaren Überschuß zur Ziel-DNA vorhanden, führen diese zwei wiederholbaren Schritte zu einem Amplifikationszyklus. Falls keine DNA-Zielsequenz zugegen war, können keine RNA-DNA-Hybride entstehen und die RNA-Moleküle werden nicht durch RNase H abgebaut.

Nachzuweisen sind also entweder die Abbauprodukte der RNA-Moleküle oder eine Abnahme der ursprünglich eingesetzten Menge an RNA-Molekülen. Der Nachweis der Abbauprodukte der zuvor markierten RNA-Moleküle beinhaltet üblicherweise gelelektrophoretische Techniken. Hierbei wird von einer erhöhten Wanderungsgeschwindigkeit der markierten RNA-Abbauprodukte oder von einer Abnahme der Intensität derjenigen Bande, welche die intakte RNA darstellt, auf die Anwesenheit der Ziel-DNA im "Cycling Probe"-Ansatz rückgeschlossen.

Die Verwendung von erfindungsgemäßen RNA-Molekülen mit zwei unterschiedlichen nicht-radioaktiven, voneinander trennbaren Markierungen stellt eine Weiterentwicklung des Systems dar. Die erste der beiden Markierungen, die sich vorzugsweise im Bereich des 5'-Endes des RNA-Moleküls befindet, kann zur Bindung der RNA an eine Festphase verwendet werden und der Nachweis des Analyten kann durch die Bestimmung der zweiten Markierung erfolgen, die sich vorzugsweise im Bereich des 3'-Endes des RNA-Moleküls befindet. Andererseits kann sich natürlich auch die erste Markierung im Bereich des 3'-Endes und die zweite Markierung im Bereich des 5'-Endes der RNA befinden. Hierbei ist anzumerken, daß die Markierungen dabei nicht auf die extremen Enden (d.h. die 5'- oder 3'-Position) beschränkt sein müssen. Beispielsweise können bei einem RNA-Molekül auch das 2. oder/und 3. Nucleotid am 5'-Ende sowie mehrere Nucleotide am 3'-Ende markiert sein.

Der Nachweis des Analyten erfolgt nun entweder durch Bestimmung der Signalabnahme der zweiten Markierung an der Festphase oder/und durch Zunahme des Signals der zweiten Markierung in der Flüssigphase bzw. an einer zweiten Festphase, die eine hochaffine Bindung mit der zweiten Markierung eingehen kann. Bevorzugte Beispiele für zwei derartige trennbare Markierungen sind Biotin und Digoxigenin oder ein anderes Hapten bzw. zwei verschiedene Haptene, z.B. Digoxigenin und Fluorescein. Die Biotinmarkierung kann an eine mit Streptavidin beschichtete Festphase binden und die Haptenmarkierung kann an eine mit Anti-(Hapten)-Antikörper beschichtete Festphase binden. Das folgende Schema zeigt die Durchführung der einzelnen Reaktionsschritte:

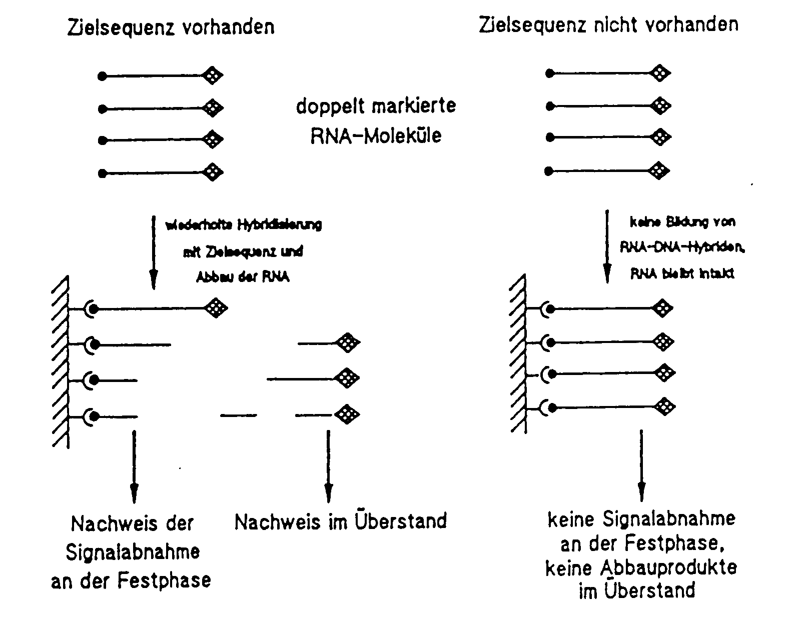

Bei diesem Vorgehen sind zum einen keine zeitaufwendigen gelelektrophoretischen Techniken nötig, und zum anderen kann der Einsatz von radioaktiven Substanzen vermieden werden. Das Prinzip dieses Verfahrens beruht darauf, daß in Anwesenheit einer Ziel-DNA im Anschluß an die Hybridisierung die RNase H durch den Abbau der RNA-Moleküle zu einer Trennung der beiden Markierungen führt. Wird nun eine dieser beiden Markierungen an eine geeignete Oberfläche gebunden, so kann bei erfolgter Trennung die Abnahme der Anzahl der anderen Markierungen an der Festphase oder/und deren Zunahme im Überstand nachgewiesen werden. Im Falle der Abwesenheit der DNA-Zielsequenz bleiben beide Markierungen miteinander verbunden und somit an eine entsprechende Oberfläche gekoppelt, so daß sich keine Abbauprodukte der RNA nachweisen lassen. Die Wahl von photometrisch auswertbaren Nachweisreaktionen und von Mikrotiterplatten (MTP) als Nachweisformat erlaubt sowohl quantitative Aussagen als auch einen hohen Probendurchsatz.

Ein Vorteil der Verwendung des erfindungsgemäßen Verfahrens beim Nachweis von Nucleinsäuren besteht auch darin, daß eine nachträgliche Markierung von in vivo erzeugter RNA möglich ist, z.B. mit einer Biotinmarkierung, so daß die auf diese Weise markierten RNA-Moleküle als Sonden zum Nachweis von Nucleinsäure-Analyten eingesetzt werden können. Die erfindungsgemäßen RNAs können im übrigen neben der Verwendung bei der Cycling-Probe-Methode aber auch als Sonden bei anderen Nucleinsäure-Nachweisverfahren, z.B. in einer direkten Hybridisierung oder in einer Sandwich-Hybridi-sierung, eingesetzt werden.

Eine weitere Anwendung der erfindungsgemäßen RNA-Moleküle ist die Isolierung spezifischer DNA-Sequenzen. So können erfindungsgemäße Sonden zum "Fischen" von spezifischen Nukleinsäuren hergestellt werden, wobei die Sonden z.B. durch eine Biotinmarkierung an eine Streptavidin-beschichtete Festphase gebunden und nach Hybridisierung mit der "gefischten" DNA-Sequenz z.B. durch alkalischen Abbau degradiert werden können. Dadurch ist "gefischte" DNA in Lösung und steht für weitere Versuche zur Verfügung.

Eine weitere Möglichkeit der Verwendung der erfindungsgemäßen RNA ist die Isolierung und Reinigung von RNA. So können durch Anfügen eines nicht-radioaktiv modifizierten Nucleotids am 3'-Ende isolierter RNA-Moleküle diese durch Bindung an eine geeignete Festphase (z.B. Streptavidin-, Anti-Digoxigenin-Antikörperbeschichtung) gereinigt werden. Durch Anfügen einer Serie gleicher Nucleotide (z.B. einem G-oder C-Tail) kann RNA selbst ohne eingeführte Markierungsgruppen über diesen Tail spezifisch aufgereinigt werden. Darüber hinaus erlaubt ein G- oder C-Tail eine cDNA-Synthese mit einem entsprechenden komplementären Primer, wobei dieses Konstrukt stabiler als bisher verwendete Konstrukte mit einem Poly-(A)-Tail und Oligo (dT)-Primern ist.

Isolierte RNA mit unbekannter Sequenz kann daher zur Bindung eines komplementären Primers für die cDNA-Synthese mit einem Tail versehen werden und durch Einbau von Markierungen kann der Tail überprüft werden und ein zu diesem Tail komplementäres Oligonucleotid kann als Primer für die cDNA-Synthese dienen. In einer Abwandlung dieses Verfahrens kann durch eine kurze Tailingzeit mit einem Nucleotid (z.B. dCTP) und anschließendes Tailing mit einer erhöhten Konzentration des komplementären Nucleotids (z.B. dGTP) die Rückfaltung des entstehenden PolyC-PolyG-Tails ausgenutzt werden, um ohne Einsatz eines zusätzlichen Primers einen cDNA-Strang mit Reverser Transkriptase zu synthetisieren.

Auch die verbesserte Isolierung spezifischer RNA-Sequenzen durch subtraktive oder differenzielle Hybridisierung ist möglich. Hierzu kann durch nachträgliche Modifizierung (z.B. mit Biotin- oder Digoxigenin-markierten Nucleotiden) der Kontroll-RNA mittels TdT diese an eine Festphase gebunden werden, die zu untersuchende RNA kann in eine stabilere cDNA umgeschrieben werden (Umkehrung der in der Literatur beschriebenen Standard-Vorgehensweise), die zur Auffindung der differenziellen Sequenzen mit der gebundenen RNA hybridisieren kann.

Das erfindungsgemäße Verfahren kann auch zur Reinigung von RNA-bindenden Proteinen verwendet werden. Hierzu wird die an der Proteinbindung beteiligte RNA 3'-terminal markiert und dann zur Bindung mit Proteinen eingesetzt, durch anschließende Bindung an eine Festphase können die RNA-Komplexe und damit die Proteine isoliert werden.

Noch eine weitere Anwendungsmöglichkeit ist die RNA-Sequenzierung. Durch Anfügung von nur einem Nucleotid an das 3'-Ende einer zu sequenzierenden RNA werden die Voraussetzungen für eine Sequenzierung durch basenspezifische Spaltung vom 3'-Ende her geschaffen. Weiterhin kann die markierte RNA immobilisiert werden und somit eine Festphasensequenzierung nach der Kettenabbruchmethode durchgeführt werden.

Noch eine weitere Anwendung besteht in der Herstellung eines pharmazeutischen Mittels, z.B. für die Antisense-Technologie, wo erfindungsgemäße 3'-modifizierte RNA-Moleküle eine diagnostische und therapeutische Anwendung finden können.

Schließlich betrifft die vorliegende Erfindung noch eine Terminale Desoxynucleotidyltransferase, die im wesentlichen RNasefrei ist und deren Verwendung zum Anfügen von Desoxynucleotiden, insbesondere von nicht-radioaktiv markierten Desoxynucleotiden an RNA-Moleküle.

Eine im wesentlichen RNase-freie Terminale Desoxynucleotidyltransferase kann Bestandteil eines Reagenzienkits zum Anfügen von Desoxynucleotiden an das 3'-Ende von RNA-Molekülen sein. Die terminale Transferase liegt vorzugsweise in einer Volumenaktivität von 10 bis 50 x 10³ U/ml in einem 50 % Glycerinpuffer, pH 6,5 vor. Weitere Bestandteile des Puffers sind vorzugsweise ein Thiolreagenz, z.B. 2-Mercaptoethanol in einer Konzentration von 1 bis 10 mmol/I, NaCI in einer Konzentration von 150 bis 250 mmol/I, Natriumcacodylat in einer Konzentration von 150 bis 250 mmol/I und EDTA in einer Konzentration von 0,1 bis 2 mmol/I. In einem derartigen Puffersystem ist die Terminale Transferase bei -20 _{°} C stabil.

Weiterhin wird die Erfindung durch die folgenden Beispiele, und Figuren und Sequenzprotokolle erläutert.

Es zeigen:
Figur 1: das Autoradiogramm einer Markierungsreaktion mit einem synthetischen Oligoribonucleotid,
Figur 2: die Sensitivität des Nachweises markierter RNA-Moleküle,
Figur 3: die Sensitivität des Nachweises eines DNA-Oligonucleotides in einer Cycling-Probe-Reaktion,
Figur 4: die Sensitivität des Nachweises eines DNA-Oligonucleotids in einer Cycling-Probe-Reaktion mit einer Streptavidin- und einer Anti-Digoxigenin-beschichteten Mikrotiterplatte,
Figur 5 die Spezifität des Nachweises unterschiedlicher DNA-Oligonucleotide.

SEQ ID NO. 1-4: die Nucleotidsequenzen der in Beispiel 1 und 2 verwendeten Oligonucleotide.

### Beispiele

### Beispiel 1

### Nicht-radioaktive Markierung von RNA-Molekülen

Die Bedingungen zum Anfügen eines Tails von nicht-radioaktiv markierten Nucleotiden an das 3'-Ende von RNA waren wie folgt: 1 ug Oligoribonucleotid (TdT 1: 5'-CGCCGCGUCGCAGAAGAUCUCAAUC-3' entsprechend 121 pmol) wurde mit 25 U TdT (Boehringer Mannheim) in 200 mmol/I Kaliumcacodylat, 25 mmol/I Tris/HCI (pH 6,6), 0,25 mg/ml Rinderserumalbumin, 5 mmol/I CoCl₂ und 0,5 mmol/I Desoxynucleotidtriphosphat (Boehringer Mannheim) in einem Endvolumen von 30 µl für 30 Minuten bei 37 °C inkubiert. Das Anfügen von nur einem Nucleotid an das 3'-Ende des Oligoribonucleotids erfolgte unter Verwendung von 0,5 mmol/I Digoxigenin (DIG)-11-ddUTP oder Biotin-16-ddUTP. Für kurze Tails wurde 0,5 mmol/I DIG-11- dUTP oder Biotin-16-dUTP oder eine Kombination von DIG-11-dUTP und DIG-11-ddUTP (jeweils 0,25 mol/I) verwendet. Für längere Tails wurde eine Nucleotidmischung verwendet, die 0,45 mmol/I dATP und 0,05 mmol/l DIG-11-dUTP enthielt. 40 U RNase-Inhibitor (Boehringer Mannheim) wurden zugegeben, da TdT möglicherweise noch eine gewisse RNase-Restaktivität enthalten kann. Die Reaktion wurde durch Zugabe von EDTA gestoppt. Für die anschließende Ethanolpräzipitation wurde Glycogen als Träger zugesetzt.

In analogen Reaktionen wurde 1 µg MS2 RNA, tRNA aus E.coli oder eine Mischung von in vitro Transkripten (300 - 1600 Basen, RNA Molekulargewicht Marker III) (alle von Boehringer Mannheim) mit TdT und 0,5 mmol/l DIG-dUTP, wie oben beschrieben, getailt.

Zur molekularen Analyse wurden die Oligonucleotide vor der TdT-Reaktion mit (y³²P)ATP und Polynucleotidkinase radioaktiv 5'-endmarkiert. Nach der 3'-Tailingreaktion wurden die Produkte auf einem 12 % Polyacrylamid-8M Harnstoff -Sequenziergel aufgetrennt und anschließend autoradiographisch analysiert. Der Nachweis des Einbaus einer nicht-radioaktiven Markierung erfolgte mittels Anti-DIG-alkische Phosphatase bzw. Streptavidin-alkalische Phosphatase und Nitroblautetrazoliumsalz und 5-Brom-4-chlor-3-indolylphos- phat in dem von Schmitz et al. (Anal.Biochem. 192 (1991), 222-231) beschriebenen colorimetrischen Immunoassay.

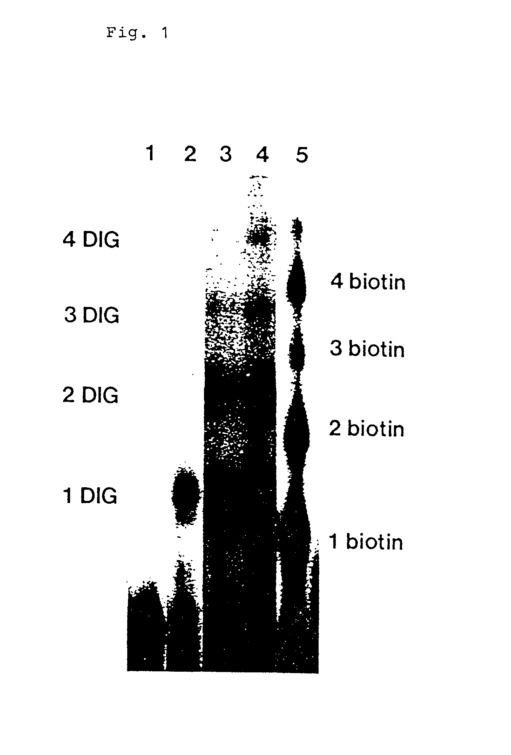

Das Ergebnis der Tailingreaktion des 5'-(³²P) Oligoribonucleotids TdT 1 ist in Fig.1 gezeigt. Spur 1 zeigt das Ribonucleotid TdT 1 ohne nicht-radioaktive Markierung, Spur 2 zeigt die Markierung mit DIG-ddUTP, Spur 3 zeigt die Markierung mit DIG-dUTP für 15 Minuten, Spur 4 die Markierung mit DIG-dUTP für 30 Minuten und Spur 5 die Markierung mit Biotin-dUTP. Unter Verwendung von DIG-dUTP konnten bei der Tailingreaktion bis zu 4 DIG-Desoxynucleotide angefügt werden. Mit Biotin-dUTP enthielten die meisten Produkte 1 bis 4 modifizierte Nucleotide, in einigen Produkten wurde noch ein fünftes Nucleotid angefügt. Eine Markierung mit Biotin-ddUTP (nicht gezeigt) oder DIG-ddUTP (Spur 1) führte zum Anfügen eines einzigen Nucleotids.

Die Markierung mit einer Nucleotidmischung aus dATP und DIG-dUTP führte zu Taillängen von bis zu etwa 50 Nucleotiden. Nach etwa 30 minütiger Inkubation war die Tailingreaktion beendet und das Produktmuster blieb für die nächsten 2 Stunden gleich.

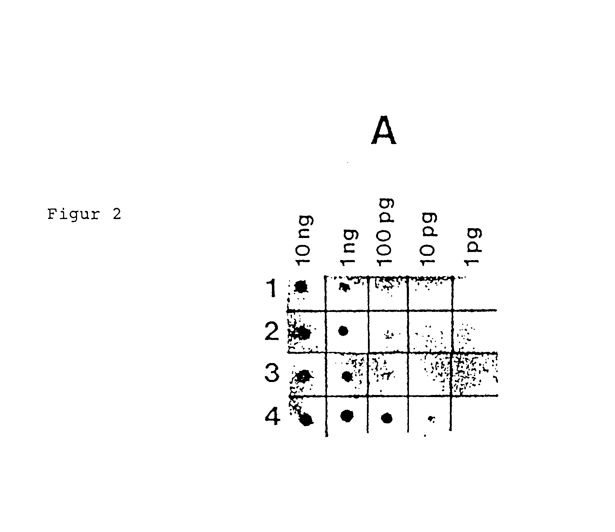

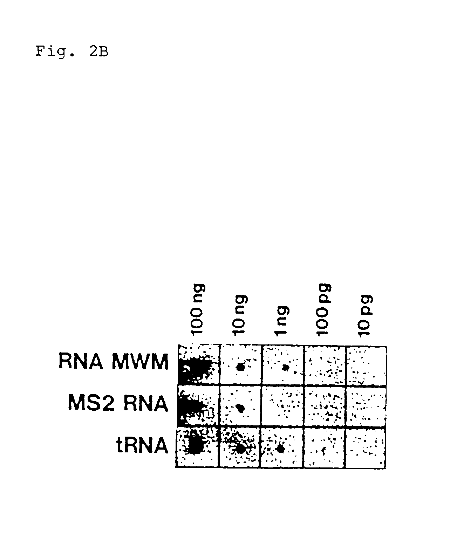

In Fig. 2 ist eine weitere Serie von Experimenten gezeigt, bei denen am 3'-Ende DIG-markierte Oligonucleotide und Polynucleotide in einer 1:10 Verdünnungsserie auf eine Membran aufgebracht wurden.

Fig. 2a zeigt die Sensitivität des Nachweises des markierten Oligoribonucleotids TdT 1 nach Reaktion in Gegenwart von TdT mit verschiedenen Nucleotidzusammensetzungen:
1. 0,5 mmol/l DIG-ddUTP,
2. 0,25 mol/l DIG-ddUTP und 0,25 mmol/l DIG-dUTP,
3. 0,5 mmol/l DIG-dUTP,
4. 0,05 mmol/l DIG-dUTP und 0,45 mmol dATP.

Fig. 2b zeigt die Nachweisgrenzen für längere RNA-Moleküle, die mit 0,5 mmol/l DIG-dUTP getailt sind (MWM: Molekulargewichtsmarker, bestehend aus in vitro Transkripten).

Wie aus Fig. 2a ersichtlich ist, hängt die Nachweisgrenze des markierten 25 Basen langen Oligoribonucleotids TdT 1 von dem bei der Tailingreaktion verwendeten Nucleotid oder Nucleotidgemisch ab. Bei DIG-ddUTP, das nur das Anfügen von einem einzigen DIG-Molekül erlaubt, war die Nachweisgrenze 1 ng. Eine äquimolare Mischung von DIG-ddUTP und DIG-dUTP (jeweils 0,25 mmol/I) oder die Zugabe von DIG-dUTP als einziges Nucleotid (wodurch das Anfügen von bis zu 4 DIG-Molekülen ermöglicht wird) erhöht die Sensitivität auf 100 pg. Das mit DIG-dUTP und dATP in einem Molverhältnis von 1:9 getailte Oligoribonucleotid zeigte eine Sensitivität von 10 pg (1,2 fmol).

Aus Fig. 2b ist ersichtlich, daß auch in vitro erzeugte RNA-Transkripte mit einer Länge von 300 - 1600 Nucleotiden (RNA-MWM), natürliche tRNA aus E.coli und virale MS2-RNA mit TdT und Biotin- oder DIG-dUTP getailt werden können. Dazu wurde jeweils 1 µg der RNA mit TdT und 0,5 mmol/l DIG-dUTP unter den oben beschriebenen Reaktionsbedingungen inkubiert. Die Ansätze wurden mit Proteinase K behandelt, um falsch positive Signale von Komplexen zwischen Enzym und markierten Nukleotiden auszuschließen. Nicht eingebaute Nukleotide wurden mittels Präzipitation der Reaktionsprodukte entfernt und letztere dann in Verdünnungsreihen auf eine Membran aufgebracht. Durch die TdT angehängte DIG-dUMP-Reste wurden wie oben beschrieben nachgewiesen. Die Nachweisgrenzen der mit DIG-dUTP als einzigem Nucleotid getailten RNA-Moleküle wurden bestimmt. Dabei wurde festgestellt, daß 100 pg (3,9 fmol) tRNA, 1 ng (3,9 fmol) von in vitro synthetisierten Transkripten und 10 ng (8,5 fmol) MS2-RNA nachgewiesen werden konnten. Die Unterschiede in der Sensitivität können möglicherweise auf die Sekundärstruktur der RNA-Moleküle, insbesondere im Bereich des 3'-Hydroxylendes, zurückzuführen sein.

### Beispiel 2

Jeweils 1 ng oder 10 ng des chemisch 5'-Biotin(BIO) - und mittels TdT 3'-DIG-markierten RNA-Oligonucleotids "HBV1" (5'-BIO-cgccgcgucgcagaagaucucaauc-(DIG)₁₋₄-3') wurden mit unterschiedlichen Mengen der nachzuweisenden DNA-Oligonucleotide "DHBV1" (5'-TTGAGATCTTCTGCGACGCGG-3': zu HBV1 komplementäre Sequenz), "MM1 DHBV1" (5'-TTGAGATCTTATGCGACGCGG-3': zu HBV1 komplementäre Sequenz mit einer zentralen Basefehlpaarung) und "MM3DHBV1 (5'-TTGAGATCTCACGCGACGCGG-3': zu HBV1 komplementäre Sequenz mit drei zentralen Basefehlpaarungen) in einem Volumen von 30 µl unter folgenden Bedingungen inkubiert:
1 ng bzw. 10 ng 5'-BIO-HBV1-DIGTail-3'
variabel nachzuweisendes DNA-Oligonucleotid
10 mmol/I HEPES pH 8,0
30 mmol/I NaCl
1 mmol/I MnCl₂
40 U RNase Inhibitor aus Plazenta (Boehringer Mannheim)
7 µg Rinderserumalbumin (RSA) (Boehringer Mannheim, Qualität für Molekularbiologie)
1 µg tRNA (Boehringer Mannheim, Gesamt-tRNA aus E.coli)
4 U RNase H (Boehringer Mannheim)

Der Ansatz wurde mit 30 µl Mineralöl überschichtet und für 3 h bei 56 °C inkubiert. Anschließend wurde die Reaktionsmischung mit 180 µl Verdünnungspuffer (10 mmol/I HEPES pH 8,0, 30 mmol/I NaCI, 1 mmol/l MnCl₂) auf ein Gesamtvolumen von 210 µl aufgefüllt; die die obere Phase bildenden 30 µl Mineralöl störten die weitere Vorgehensweise nicht. Aus der unteren wäßrigen Phase wurden je zwei mal 100 µl in eine Streptavidin(SA)-beschichtete Mikrotiterplatte (MTP) (Boehringer Mannheim), die mit Waschpuffer (PBS, 0,5 % (v/v) Tween 20) vorgewaschen war, pipettiert und der Abbau des RNA-Oligonucleotids in Gegenwart des komplementären DNA-Oligonucleotides folgendermaßen nachgewiesen:

Die SA-MTP wurde unter Schütteln (Well-Warm 1, Fa. Denley Instruments GmbH) für 1 h bei 37°C inkubiert, um eine Bindung des an die RNA gekoppelten Biotins an Streptavidin zu ermöglichen. Nach erfolgter Anlagerung wurde der Inhalt von jeweils 2 Vertiefungen ("Überstand") entsprechend den Doppelbestimmungen vereinigt und für die unten beschriebene Detektion in einer mit Anti-Digoxigenin-Antikörper beschichteten Mikrotiterplatte (<DIG>-MTP) eingesetzt. Die SA-MTP wurde fünfmal mit je 200 µl Waschpuffer gewaschen. Anschließend wurden je 100 µl einer (DIG)-Peroxidase (POD)_{poly-}Konjugatverdünnung (in Konjugatpuffer: 100 mmol/l Na-Phosphat pH 7,5, 0,9 % NaCI, 1 % Rafulon F4J oder RSA Fraktion V, mit Diethylpyrocarbonat behandelt, sterilfiltriert, bei + 4°C aufbewahrt) zugegeben und erneut unter denselben Bedingungen inkubiert. Ungebundene Konjugatmoleküle wurden durch fünfmaliges Waschen entfernt. Pro Vertiefung wurden nun 100 µl der Substratlösung (2,2'-Azino-di-[3-ethylbenzthiazolinsulfonat(6)], ABTS) zugegeben; die Farbreaktion erfolgte bei 37°C unter Schütteln. Die "Optische Dichte" des umgesetzten ABTS bei 405 nm wurde mittels eines ELISA-Readers (SLT) gemessen und nach Abzug des Nullwertes (nur ABTS) die Mittelwerte der Doppelbestimmungen ermittelt. Als Negativkontrolle dienten Ansätze, die keine DNA-Oligonucleotide enthielten.

Der Nachweis von degradierten RNA-Molekülen im Überstand erfolgte in einer (DIG)-MTP. Hierfür wurden die nach Inkubation in der SA-MTP gewonnenen Überstände eingesetzt: Jeweils zwei mal 90 µl wurden in die mit Waschpuffer vorgewaschene (DIG)-MTP pipettiert und die sich anschließenden Schritte zur Detektion wie für die SA-MTP beschrieben durchgeführt.

### Sensitivität des Nachweises

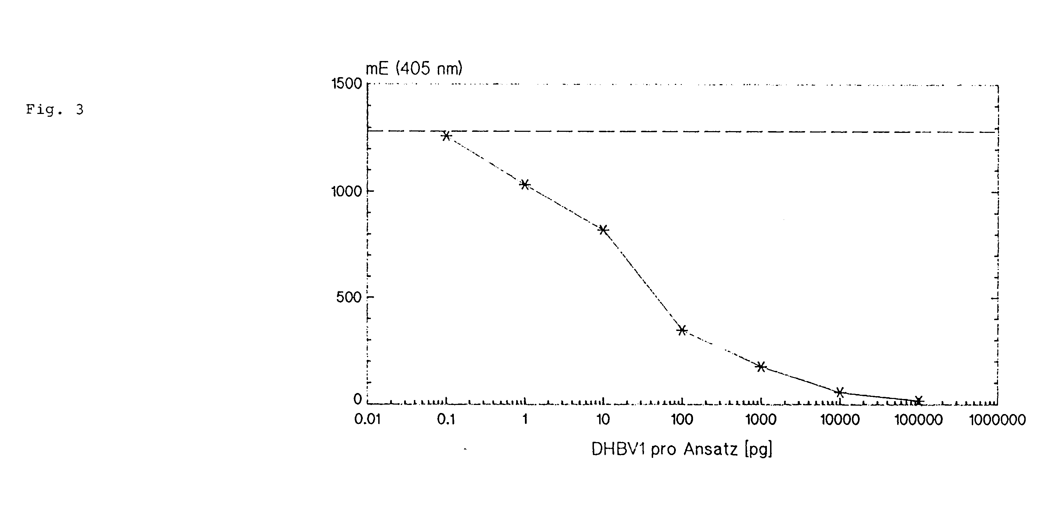

In einer 1:10-Verdünnungsreihe wurden jeweils zwischen 0,1 pg und 100 ng des DNA-Oligonucleotids "DHBV1 " in die Nachweisreaktion mit 1 ng des RNA-Oligonucleotids HBV1 eingesetzt. Die Detektion in der SA-MTP lieferte die in Fig.3 graphisch dargestellte Werte. Dort ist die "Optische Dichte" (in relativen Einheiten) in Abhängigkeit von der nachzuweisenden DNA-Menge (in pg pro Ansatz) angegeben. Der gemessene Wert der Negativkontrolle (Ansatz ohne "DHBV1 ") wurde als waagrechte Linie dargestellt.

Mit zunehmender Menge an Ziel-DNA wurden mehr RNA-Oligonucleotide durch wiederholte Hybridisierung und Degradation abgebaut. Diesen Abbau spiegelt die Verringerung des erhaltenen Signals wider. Mittels eines solchen Ansatzes lassen sich 1 pg (0,121 fmol) eines DNA-Oligonucleotids nachweisen. Auch der Zusatz von 10 µg Kalbsthymus-DNA pro Ansatz führte zu einem vergleichbaren Kurvenverlauf.

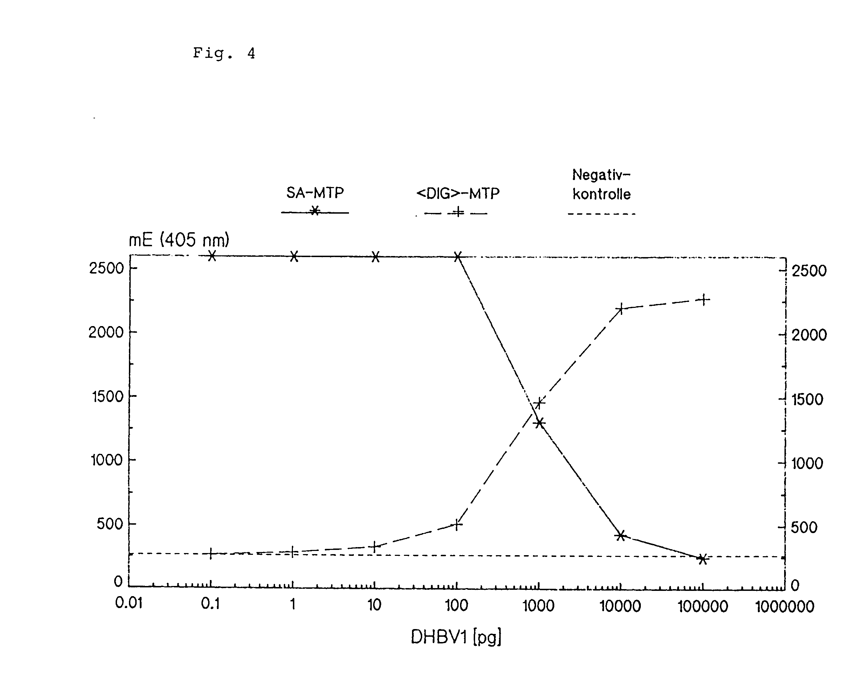

Der Nachweis im Überstand in (DIG)-MTP zeigte eine umgekehrte Abhängigkeit des Signals von der DNA-Menge: Mit zunehmender DNA-Menge stieg das Signal an. Da die erhaltenen Werte niedriger als die in der SA-MTP erhaltenen Werte waren, wurden 10 ng 5'-BIO-HBV1-DIGTail-3' in die Nachweisreaktion eingesetzt. Die Abhängigkeit des Signals von der Menge an DNA ist für die Detektion in SA-MTP und in (DIG)-MTP in Fig.4 graphisch dargestellt. Dort ist die "Optische Dichte" (in relativen Einheiten) in Abhängigkeit von der nachzuweisenden DNA-Menge (in pg pro Ansatz) angegeben. Der gemessene Wert der Negativkontrolle (Ansatz ohne "DHBV1 ") für die (DIG)-MTP wurde als waagrechte Linie dargestellt; für die SA-MTP ergaben sich für die Negativkontrolle sowie für die Ansätze mit 0,1 bis 100 pg "DHBV1" Werte, die den meßbaren Bereich überschritten.

Der Nachweis in der (DIG)-MTP zeigt eine etwa um den Faktor 100 geringere Sensitivität im Vergleich zum Nachweis in der SA-MTP.

### Spezifität des Nachweises

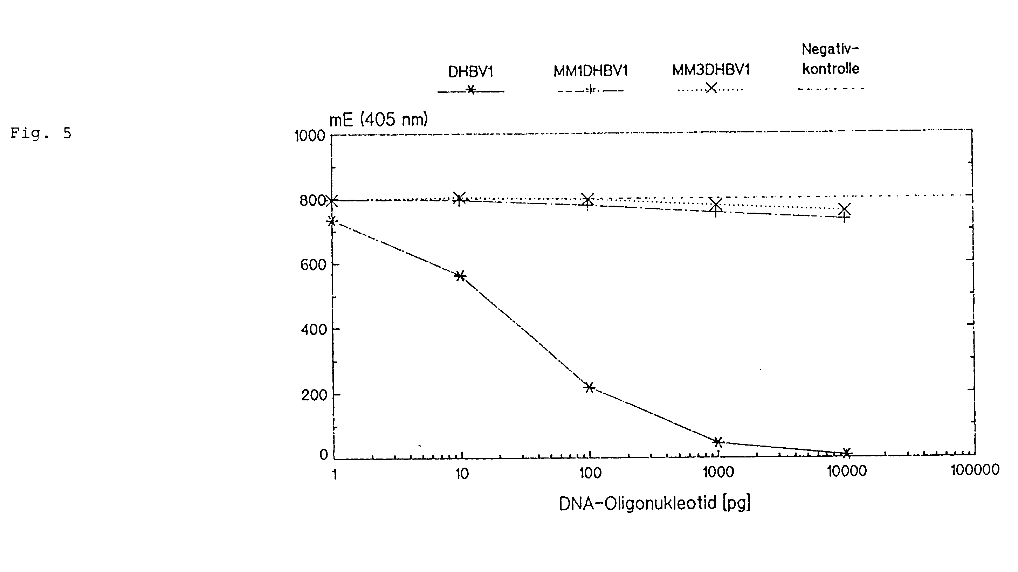

Die Untersuchung der Spezifität wurde anhand von drei verschiedenen DNA-Oligonucleotiden durchgeführt, die entweder perfekt komplementär ("DHBV1") zu "HBV1 waren oder an zentraler Position einen "MM1 DHBV1 ") oder drei ("MM3DHBV1 ") Basenfehlpaarungen aufwiesen. Nur bei perfekter Komplementarität beider Oligonucleotide kann unter diesen Reaktionsbedingungen das entsprechende DNA-Oligonucleotid nachgewiesen werden (Fig.5). Dort ist die "Optische Dichte" (in relativen Einheiten) in Abhängigkeit von der nachzuweisenden DNA-Menge (in pg pro Ansatz) angegeben. Der gemessene Wert der Negativkontrolle (Ansatz ohne DNA-Oligonucleotid) wurde als waagrechte Linie dargestellt.

Wie aus anderen Experimenten hervorging, beruhte die Spezifität auf den stringenten Hybridisierungsbedingungen. Diese wurden allerdings so gewählt, daß die RNase H unter solchen Bedingungen noch aktiv war. Bei Verringerung der Inkubationstemperatur oder Erhöhung der lonenstärke im Inkubationspuffer ließ sich auch "MM1 DHBV1 " und - bei weiterer Verminderung der Stringenz - "MM3DHBV1 " nachweisen. Die dabei ermittelte Sensitivität entsprach der in Fig.3 dargestellten Sensitivität.

## Patentansprüche

1. Verfahren zum Einführen von Nucleotiden, die eine nicht-radioaktive Markierungsgruppe tragen, in Nucleinsäuren,
dadurch gekennzeichnet,
daß man mindestens ein nicht-radioaktiv markiertes Desoxynucleotid an das 3'-Ende eines Nucleinsäure-Akzeptormoleküls mit mindestens einem 3'-terminalen Ribonucleotid durch die Terminale Desoxynucleotidyltransferase (EC 2.7.7.31) anfügt.

2. Verfahren nach Anspruch 1,
dadurch gekennzeichnet,
daß man als Nucleinsäure-Akzeptormolekül eine Ribonucleinsäure verwendet.

3. Verfahren nach einem der Ansprüche 1 oder 2,
dadurch gekennzeichnet,
daß man als Desoxynucleotide 2'-Desoxynucleosidtriphosphate oder/und 2',3'-Didesoxynucleosidtriphosphate verwendet.

4. Verfahren nach einem der Ansprüche 1 bis 3,
dadurch gekennzeichnet,
daß man Desoxynucleotide verwendet, die eine Fluoreszenz-, Chemilumineszenz-, NMR-aktive oder/und zur hochaffinen Bindung mit einem Reaktionspartner fähige Markierungsgruppe tragen.

5. Verfahren nach einem der Ansprüche 1 bis 4,
dadurch gekennzeichnet,
daß man die Reaktion in Anwesenheit eines RNase-Inhibitors durchführt.

6. Verfahren nach einem der Ansprüche 1 bis 5,
dadurch gekennzeichnet,
daß man die Reaktion mit einer im wesentlichen RNase-freien Terminalen Desoxynucleotidyltransferase durchführt.

7. Verfahren zum Einführen von Markierungsgruppen in RNA-Moleküle,
dadurch gekennzeichnet,
daß man mindestens ein Desoxynucleotid, das eine Markierungsgruppe trägt, durch die Terminale Desoxynucleotidyltransferase (EC 2.7.7.31) an das 3'-Ende der RNA-Moleküle anfügt.

8. RNA-Molekül, das im Bereich seines 3'-Endes mindestens ein Desoxynucleotid enthält, das eine nicht- radioaktive Markierungsgruppe trägt, wobei die Markierung kovalent an die Nucleotidbase des Desoxynucleotids gebunden ist.

9. Verwendung eines RNA-Moleküls nach Anspruch 8 oder eines gemäß dem Verfahren nach einem der Ansprüche 1 bis 7 hergestellten RNA-Moleküls als Sonde zum Nachweis von Nukleinsäureanalyten.

10. Verwendung nach Anspruch 8 in einem Cycling-Probe-Verfahren, das die Schritte umfaßt:
(a) Inkontaktbringen einer Probeflüssigkeit mit zu einem Abschnitt eines DNA-Analyten komplementären, markierten RNA-Molekülen unter solchen Bedingungen, daß bei Anwesenheit des Analyten eine Hybridisierung mit den markierten RNA-Molekülen stattfindet,
(b) Behandeln der Probeflüssigkeit mit RNase H unter solchen Bedingungen, daß bei Vorhandensein eines RNA-DNA-Hybrids ein Abbau der RNA-Moleküle im Hybrid und eine Freisetzung der abgebauten RNA-Bruchstücke stattfindet,
(c) gegebenenfalls ein- oder mehrmaliges Wiederholen der Schritte (a) und (b) und
(d) Nachweisen des Vorhandenseins oder des Fehlens des DNA-Analyten durch Bestimmung der abgebauten RNA-Bruchstücke oder/und der nicht abgebauten RNA-Moleküle.

11. Terminale Desoxynucleotidyltransferase, die im wesentlichen frei von einer RNase-Aktivität ist.

12. Reagenzienkit zum Anfügen von Desoxynucleotiden an das 3'-Ende von RNA-Molekülen, dadurch gekennzeichnet,
daß er eine Terminale Desoxynucleotidyltransferase nach Anspruch 11 enthält.
